(19) **Europäisches Patentamt / European Patent Office / Office européen des brevets**

(11) **EP 4 756 730 A1**

(12) # EUROPEAN PATENT APPLICATION

(43) Date of publication:
**10.06.2026 Bulletin 2026/24**

(21) Application number: **25219651.4**

(22) Date of filing: **01.12.2025**

(51) International Patent Classification (IPC):
$G06V\ 10/46^{(2022.01)}$    $G06V\ 10/62^{(2022.01)}$
$G06V\ 10/25^{(2022.01)}$    $G06V\ 10/44^{(2022.01)}$
$G06V\ 10/764^{(2022.01)}$    $G06V\ 10/774^{(2022.01)}$
$G06V\ 10/82^{(2022.01)}$    $G06V\ 20/20^{(2022.01)}$
$G06V\ 20/60^{(2022.01)}$    $G06V\ 40/10^{(2022.01)}$
$G06V\ 40/20^{(2022.01)}$

(52) Cooperative Patent Classification (CPC):
**G06V 10/25; G06V 10/454; G06V 10/764;
G06V 10/774; G06V 10/82; G06V 20/20;
G06V 20/60; G06V 40/103; G06V 40/23;**
G06V 10/46; G06V 10/62; G06V 2201/06

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **04.12.2024 KR 20240178492**

(71) Applicant: **Kyungpook National University
Industry-Academic
Cooperation Foundation
Daegu 41566 (KR)**

(72) Inventors:
• **KIM, Bub Ruyr**
  **Daegu 41566 (KR)**
• **LEE, Dong Eun**
  **Daegu 41566 (KR)**
• **MI, Yi Rong**
  **Daegu 41566 (KR)**

(74) Representative: **AWA Sweden AB
Box 5117
200 71 Malmö (SE)**

(54) **METHOD AND SYSTEM FOR DEEP LEARNING-BASED MUSCULOSKELETAL DISEASE PREDICTION**

(57) The present invention relates to a method and system for deep learning-based musculoskeletal disease prediction. A method for deep learning-based musculoskeletal disease prediction, according to the present invention, may comprise: receiving an image of a worker serving as a photographing target and a specific environment in which the worker is positioned; specifying, from the image, a first bounding-box corresponding to the worker and a second bounding-box corresponding to the specific environment by using a pre-trained deep learning model; extracting keypoints corresponding to a plurality of joints included in a body of the worker based on the first bounding-box; calculating angles between the plurality of joints by using the extracted keypoints and calculating a risk score on the calculated angles between the plurality of joints; specifying a risk degree for the worker based on the calculated risk score; and generating response action information according to the specified risk degree.

EP 4 756 730 A1

# EP 4 756 730 A1

**Description**

CROSS REFERENCE TO RELATED APPLICATION

The present application claims priority to Korean Patent Application No. 10-2024-0178492, filed December 4, 2024, the entire contents of which are hereby incorporated by reference in its entirety.

BACKGROUND OF THE INVENTION

Field of the Invention

The present invention relates to a method and system for deep learning-based musculoskeletal disease prediction.

Description of the Related Art

A musculoskeletal disease refers to pain or injuries occurring in the musculoskeletal system, including muscles, nerves, tendons, ligaments, bones, surrounding tissues, and the like. A musculoskeletal disease appears in various parts of the human body, such as the neck, back, arms, legs, etc.

A musculoskeletal disease occurs when specific body parts are overused due to incorrect working postures and repetitive tasks. In particular, in the construction industry, workers may impose considerable strain on the musculoskeletal system by lifting heavy objects, performing repetitive tasks, or maintaining uncomfortable postures for long periods.

In this regard, according to a report by the World Health Organization (WHO), the economic losses due to the musculoskeletal disease are shown to be very high. That is, such musculoskeletal disease is chronic pain that affects not only daily life but also economic activities.

Recently, various working posture evaluation methods have been provided as methods for evaluating working posture, such as self-report, direct measurement, and observation evaluation methods. However, the self-report method may be subjective and may have low reliability, and direct measurement may interfere with work due to sensor attachment.

Therefore, to solve these problems, there is a need for a method that utilizes deep learning technology to analyze the worker's posture and automatically evaluate the risk level.

SUMMARY OF THE INVENTION

The present invention is directed to providing a method and system for deep learning-based musculoskeletal disease prediction that is capable of predicting potential musculoskeletal disease that may occur due to various incorrect postures.

More specifically, the present invention is directed to providing a method and system for deep learning-based musculoskeletal disease prediction that is capable of analyzing a worker's posture and movements in real time and providing immediate feedback.

Further, the present invention is directed to providing a method and system for deep learning-based musculoskeletal disease prediction that is predicting risky postures at an early stage and flexibly respond to the predicted risky postures.

To solve the aforementioned objects, there is provided a method for deep learning-based musculoskeletal disease prediction, according to the present invention. The method may comprise: receiving an image of a worker serving as a photographing target and a specific environment in which the worker is positioned; specifying, from the image, a first bounding-box corresponding to the worker and a second bounding-box corresponding to the specific environment by using a pre-trained deep learning model; extracting keypoints corresponding to a plurality of joints included in a body of the worker based on the first bounding-box; calculating angles between the plurality of joints by using the extracted keypoints and calculating a risk score on the calculated angles between the plurality of joints; specifying a risk degree for the worker based on the calculated risk score; and generating response action information according to the specified risk degree.

Further, the image may include at least one among the worker's posture, motion, motion pattern, a body of the worker, and the specific environment, and the specific environment may include at least one among a workspace in which the worker is working and positions of tools, equipment, and a workpiece positioned in the workspace.

Further, the specifying of the bounding-box may comprise: processing the received image as an input to the deep learning model; analyzing, by the deep learning model, the worker and the specific environment from the image; and specifying the first bounding-box corresponding to the worker and the second bounding-box corresponding to the specific environment by using a result of the analysis.

Further, the extracting of the keypoints may comprise: adjusting joint positions corresponding to the plurality of joints according to a body structure of the worker based on the first bounding-box; and extracting the keypoints from the joint positions adjusted according to the body structure.

Further, the joint-angle calculation may comprise: generating vectors corresponding to the joint positions based on the extracted keypoints; and calculating joint angles between the joint positions by using the generated vectors.

Further, the risk-score calculation may comprise: predicting a risk level of the worker by using the calculated joint angles; and calculating the risk score for the joint angles based on a result of the prediction.

Further, the specifying of the risk degree may comprise: processing the risk score on the calculated joint angles as an input to the deep learning model; and specifying, by the deep learning model, the risk degree corresponding to the risk score among a plurality of risk degrees based on a preset criterion, and the plurality of risk degrees may include at least one among a first risk degree specified based on the risk score not satisfying the preset criterion, a second risk degree specified based on the risk score satisfying the preset criterion, and a third risk degree specified based on the risk score exceeding the preset criterion.

Further, different response action information may exist matched to the respective plurality of risk degrees, and the different response action information may include at least one among first response action information matched to the first risk degree, second response action information matched to the second risk degree, and third response action information matched to the third risk degree, and the generating of the response action information may comprise: generating the second response action information or the third response action information for predicting a musculoskeletal disease, when the specified risk degree corresponds to at least one of the second risk degree and the third risk degree that are different from the first risk degree.

Meanwhile, there is provided a system for deep learning-based musculoskeletal disease prediction, according to the present invention. The system may comprise: a data collection unit configured to receive an image of a worker serving as a photographing target and a specific environment in which the worker is positioned; and a control unit configured to specify a first bounding-box corresponding to the worker and a second bounding-box corresponding to the specific environment from the image by using a pre-trained deep learning model, in which the control unit may be configured to: extract keypoints corresponding to a plurality of joints included in a body of the worker based on the first bounding-box; calculate angles between the plurality of joints by using the extracted keypoints and calculate a risk score on the calculated angles between the plurality of joints; specify a risk degree for the worker based on the calculated risk score; and generate response action information according to the specified risk degree.

Meanwhile, there is provided a program stored on a computer-readable recording medium, executed by one or more processes in an electronic device, according to the present invention. The program may comprise instructions for performing: receiving an image of a worker serving as a photographing target and a specific environment in which the worker is positioned; specifying, from the image, a first bounding-box corresponding to the worker and a second bounding-box corresponding to the specific environment by using a pre-trained deep learning model; extracting keypoints corresponding to a plurality of joints included in a body of the worker based on the first bounding-box; calculating angles between the plurality of joints by using the extracted keypoints and calculating a risk score on the calculated angles between the plurality of joints; specifying a risk degree for the worker based on the calculated risk score; and generating response action information according to the specified risk degree.

As described above, according to the method and system for deep learning-based musculoskeletal disease prediction of the present invention, images of a worker and a work environment may be collected only by a camera device. Through this, in the present invention, initial installation and maintenance costs may be reduced compared to conventional sensor-based systems.

In addition, according to the method and system for deep learning-based musculoskeletal disease prediction of the present invention, joint positions may be detected by using a deep learning model, and angles between joints may be calculated to analyze a working posture. Through this, in the present invention, joint positions may be detected only from images without additional equipment, and even when a worker changes a posture or performs complex motions, the joint positions may be tracked in real time.

Further, according to the method and system for deep learning-based musculoskeletal disease prediction of the present invention, a worker's posture and movement may be analyzed in real time, and immediate feedback may be provided. Through this, a user may recognize a possibility of occurrence of a musculoskeletal disease at an early stage and may take preventive actions.

Furthermore, according to the method and system for deep learning-based musculoskeletal disease prediction of the present invention, a risky posture may be predicted at an early stage, and a worker may be supported to recognize the posture in real time. Through this, a worker may immediately correct a risky posture and maintain a safe posture, thereby preventing occurrence of a musculoskeletal disease.

BRIEF DESCRIPTION OF THE DRAWINGS

FIG. 1 is a conceptual diagram for explaining a system for deep learning-based musculoskeletal disease prediction according to the present invention.

FIG. 2 is a flowchart for explaining a method for deep learning-based musculoskeletal disease prediction.

FIGS. 3, 4, 5, and 6 are conceptual diagrams for explaining a method for deep learning-based musculoskeletal disease prediction according to the present invention.

FIGS. 7 and 8 are conceptual diagrams for explaining an embodiment of a user interface screen according to the present invention.

DETAILED DESCRIPTION OF THE INVENTION

Hereinafter, exemplary embodiments disclosed in the present specification will be described in detail with reference to the accompanying drawings. The same or similar constituent elements are assigned with the same reference numerals regardless of reference numerals, and the repetitive description thereof will be omitted. The suffixes "module", "unit", "part", and "portion" used to describe constituent elements in the following description are used together or interchangeably in order to facilitate the description, but the suffixes themselves do not have distinguishable meanings or functions. In addition, in the description of the exemplary embodiment disclosed in the present specification, the specific descriptions of publicly known related technologies will be omitted when it is determined that the specific descriptions may obscure the subject matter of the exemplary embodiment disclosed in the present specification. In addition, it should be understood that the accompanying drawings are provided only to easily understand the embodiments disclosed in the present specification, and the technical teachings disclosed in the present specification are not limited by the accompanying drawings, and includes all alterations, equivalents, and alternatives that are included in the teachings and the technical scope of the present invention.

The terms including ordinal numbers such as "first," "second," and the like may be used to describe various constituent elements, but the constituent elements are not limited by the terms. These terms are used only to distinguish one constituent element from another constituent element.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element exists between the constituent elements.

Singular expressions include plural expressions unless clearly described as different meanings in the context.

In the present application, it should be understood that terms "including", "having", and the like are intended to designate the existence of characteristics, numbers, steps, operations, constituent elements, and components described in the specification or a combination thereof, and do not exclude a possibility of the existence or addition of one or more other characteristics, numbers, steps, operations, constituent elements, and components, or a combination thereof in advance.

The present invention relates to a method and system for deep learning-based musculoskeletal disease prediction. The system for musculoskeletal disease prediction according to the present invention may be a system that predicts potential musculoskeletal disease that may occur due to various incorrect postures of a user (or worker). In addition, the system for musculoskeletal disease prediction according to the present invention may be a system that analyzes the user's posture and movement in real time and provides immediate feedback.

In one embodiment, the system for musculoskeletal disease prediction according to the present invention may improve the image capturing (or photographing) target's working posture and specific environment and may be used in high-risk work environments such as industrial sites and construction sites.

Here, musculoskeletal disease (Musculoskeletal Disorders, MSDs) may refer to diseases that include pain or injuries occurring in the tissues that make up the musculoskeletal system, such as muscles, joints, nerves, ligaments, tendons, and bones. Such musculoskeletal disease mainly occurs when excessive burden is placed on a specific part due to incorrect posture, repetitive motion, excessive force use, inappropriate work environments, and the like.

Major joints may refer to important joint points when analyzing the human body's movement and posture. For example, as major joints, individual joints such as a head, a neck, a thorax (center of the upper body), a waist (or pelvis), a left shoulder, a right shoulder, a left elbow, a right elbow, a left wrist, a right wrist, a left hip, a right hip, a left knee, a right knee, a left ankle, and a right ankle may be included.

The system for musculoskeletal disease prediction according to the present invention may include a deep learning model, and the deep learning model according to the present invention may be a model for object detection (or sensing, recognition, etc.) based on YOLOv8.

In one embodiment, a structure of a YOLOv8 model may be configured to include three major configurations of a backbone network, a neck network, and a head network.

The backbone network may mainly perform feature extraction, and may convert an input image into a multi-scale and multi-level feature map through a series of convolution layers, pooling operations, and other transformations.

YOLOv8 may apply a cross stage partial network (CSPNet) as a core module of the backbone network. CSPNet may enhance feature representation and may reduce calculation complexity by partially connecting feature maps between stages. A feature map may be separated into two parts, one part may directly perform feature extraction, and the other part may undergo cross-stage connection, after which both parts may be merged.

The neck network may be positioned between a backbone and a detection head, may integrate and enhance features generated from the backbone network, and may perform the role of complementarily combining feature maps of various resolutions. In a neck design of VOLOv8, a core technology may include C2F, which may combine features of various levels with context information and may enhance semantic feature representation.

In addition, as another technology, a path aggregation (PANet) may combine features of various resolutions and semantic levels through top-down path aggregation.

A head design may operate as an output layer of a model, and may be responsible for specific object detection tasks such as an object class, a position, and a confidence score. A head design of YOLOv8 may generally include multi-detection heads designed to process feature maps of various scales, and such heads may process input feature maps by utilizing convolution layers and fully connected layers and may generate final detection results.

Further, the head design of YOLOv8 may adopt an anchor-free method or an anchor-based method. The anchor-free method may directly predict a center point, a width, and a height of an object and may reduce calculation complexity and may increase detection speed, and the anchor-based method may detect objects of various shapes and sizes by adjusting predefined anchor boxes.

That is, the system for musculoskeletal disease prediction according to the present invention may include a deep learning model based on a YOLOv8 model in order to predict various musculoskeletal diseases that may occur at industrial or construction sites.

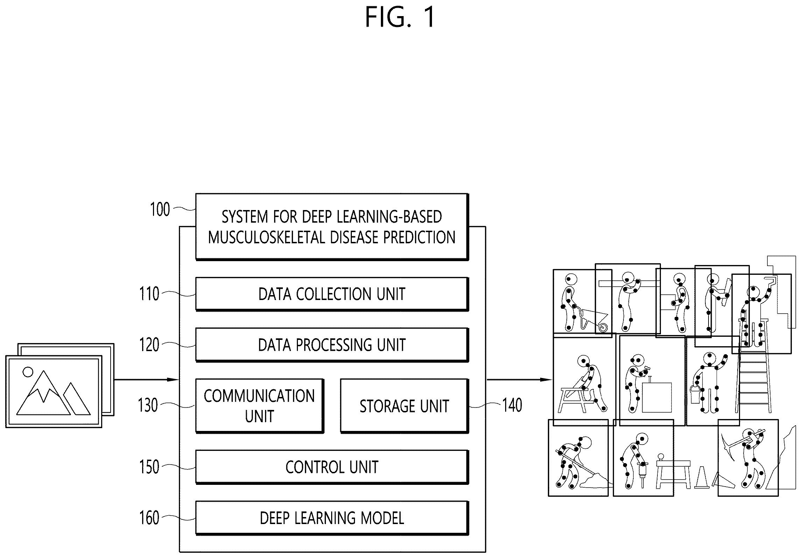

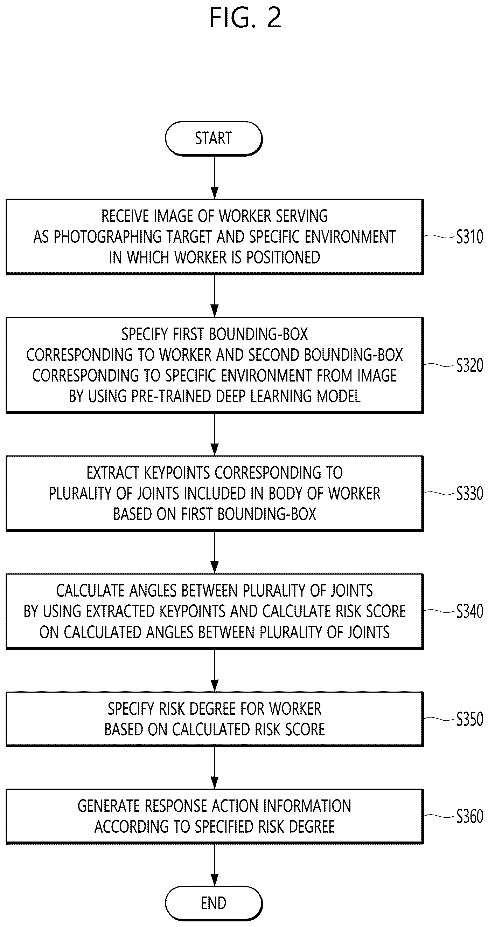

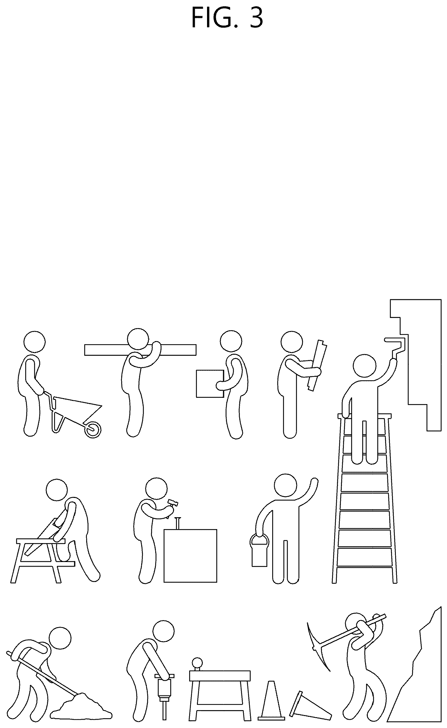

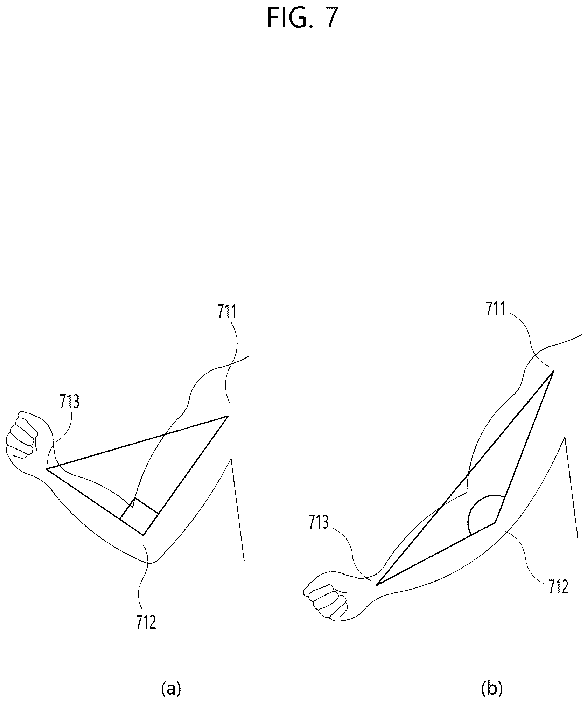

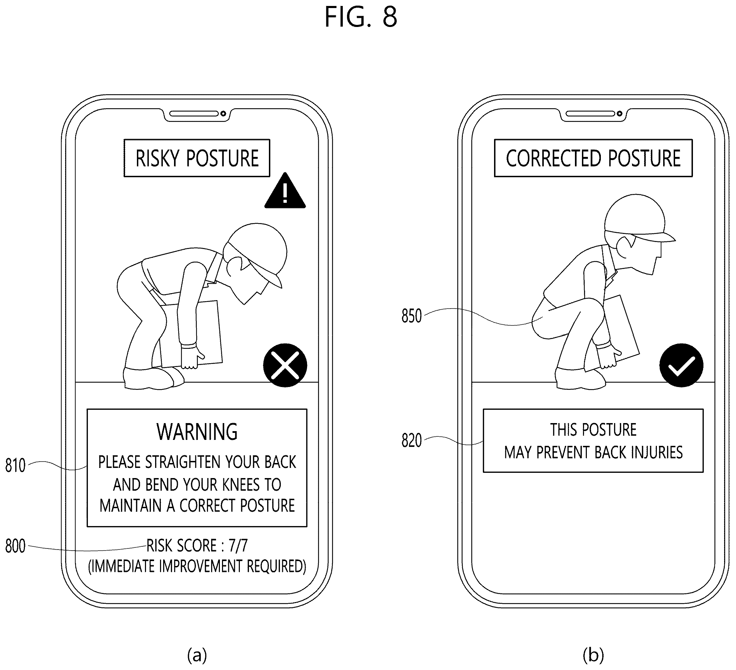

Hereinafter, the present invention will be described in more detail with reference to the accompanying drawings. FIG. 1 is a conceptual diagram for explaining a system for musculoskeletal disease prediction according to the present invention. FIG. 2 is a flowchart for explaining a method for deep learning-based musculoskeletal disease prediction, and FIG. 3, FIG. 4, FIG. 5, and FIG. 6 are conceptual diagrams for explaining a method for deep learning-based musculoskeletal disease prediction according to the present invention. Further, FIG. 7 and FIG. 8 are conceptual diagrams for explaining one embodiment of a user interface screen according to the present invention.

Meanwhile, as illustrated in FIG. 1, a system 100 for musculoskeletal disease prediction according to the present invention may include at least one among a data collection unit 110, a data processing unit 120, a communication unit 130, a storage unit 140, a control unit 150, and a deep learning model 160.

The data collection unit 110 may be configured to collect various data related to a worker serving as a photographing (or image capturing) target and a specific environment in which the worker is positioned. For example, the data collection unit 110 may collect video (or image) data related to musculoskeletal diseases at a construction (or industrial) site from various sources (for example, AI-Hub, a control system, a database, web crawling, an API, a server linked to the system 100 for musculoskeletal disease prediction, an external server, etc.). In addition, the data collection unit 110 may include at least one vision-based data collection device.

For example, the vision-based data collection device is a device that collects data by capturing visual information such as an image or a video. This device may mainly include a camera, an image sensor, image processing software, and the like, and may be used to collect visual data and analyze the visual data. The vision-based data collection device may include at least one among a digital camera, a smartphone camera, a CCTV camera, a vision system of an autonomous vehicle, an industrial vision system, smart glasses (e.g., Google Glass™), a drone, scanning equipment (e.g., a 3D scanner), and a vision-based robot system.

However, there is no particular limitation on types constituting the data collection unit 110 in the present invention, and it is only required that a function defined by each device be implemented.

The data processing unit 120 may be configured to process collected image data, convert the collected image data into image frames, specify (or select, extract, etc.) high-quality data, and then augment the data through image augmentation. In this case, the data processing unit 120 may annotate the converted image frames around an object of interest (for example, a worker, heavy equipment, a product, a transport tool, etc.).

Next, the communication unit 130 may be connected to a control system (or a control server or a control room), an apparatus (or device), an external server, one or more networks, and the like through a wireless or wired network, and may be configured to receive or transmit overall data and information necessary for an operation of the system 100 for musculoskeletal disease prediction.

Here, the control system may be configured to control workers positioned within a specific site (for example, a construction site or an industrial site), and may manage the workers by using various information (for example, response action information on a risky posture of a worker) provided by the system 100 for musculoskeletal disease prediction.

In addition, the communication unit 130 may receive an image collected from the data collection unit 110. For example, the communication unit 130 may receive motion information, position information, tool information, a risky posture, a surrounding environment, and the like of a construction-site worker from the data collection unit 110.

Further, the communication unit 130 may support various communication methods according to a communication standard of a device communicating with the communication unit 130.

For example, the communication unit 130 may be configured to communicate with a communication target using at least one of wireless LAN (WLAN), wireless-fidelity (Wi-Fi), wireless-fidelity (Wi-Fi) direct, digital living network alliance (DLNA), wireless broadband (WiBro), world interoperability for microwave access (WiMAX), high speed downlink packet access (HSDPA), high speed uplink packet access (HSUPA), long term evolution (LTE), long term evolution-advanced (LTE-A), fifth generation mobile telecommunication (5G), Bluetooth™, radio frequency identification (RFID), infrared communication (infrared data association (IrDA)), ultra-wideband (UWB), ZigBee, near field communication (NFC), or wireless universal serial bus (wireless USB) technologies.

Meanwhile, a storage unit 140 may also be referred to as a "database (DB)" or "memory", and may be configured to store various information related to the present invention. The storage unit 140 in the present invention may be provided in the system 100 for musculoskeletal disease prediction itself. In addition, at least a part of the storage unit 140 may be configured as a cloud server (or a cloud storage). That is, the storage unit 140 is sufficient to be a space where information (or data) necessary for the operation of the system 100 for musculoskeletal disease prediction according to the present invention is stored, and it may be understood that there are no constraints on physical space.

Various information related to a photographing target may be stored in the storage unit 140. For example, various information related to the photographing target may include i) photographing-target body information (joint position, an angle between joints, a body-center position, a motion pattern, etc.), ii) working-posture information (a working-posture type, identification of an improper posture, a frequency of posture changes, etc.), iii) behavior information during work (movement during work, work speed and rhythm, etc.), iv) specific-environment information (surrounding environmental conditions, a usage state of work tools, a workspace layout, etc.), additional data for risk evaluation (work-time data, work load, work-environment risk factors, etc.), v) worker-individual data (worker-body characteristics, work experience and skill level, etc.), and the like.

Specifically, information of at least one among photographing-target information, body characteristics of the photographing target (e.g., height, weight, etc.), a motion type of the photographing target, a posture-change record, a layout state of a specific environment, information on tools and equipment in use, conditions of the specific environment (e.g., space size, equipment position, etc.), an image of the photographing target and a characteristic environment captured by a vision-based data collection device, an image preprocessed for training the deep learning model 160, an augmented image (rotation, zoom-in, color adjustment, etc.), a bounding-box (boundary-box information of the photographing target and the specific environment), a keypoint (key joint-position information of the photographing target), a coordinate value (x, y) representing each joint position of the photographing target, an angle between joints and a calculation value for posture evaluation, a risk score of the photographing target calculated by the deep learning model 160, a result specifying a risk degree (low risk, medium risk, and high risk), a posture-suitability evaluation result of the photographing target, a frequency and duration of posture changes, a motion pattern of the photographing target, a frequency of repetitive motions, a posture-change history performed by the photographing target, a change in a specific environment and a change history of a layout state, weights and parameters of a trained model, a training dataset and a verification dataset, a joint-angle calculating method, a risk-score calculation standard, a system-operation setting value, a posture analysis of the photographing target, a change in a risk score, an improvement-action record, a state analysis of a specific environment, an improvement suggestion, a risk-score change graph, and statistics of posture analysis may be matched and stored in the storage unit 140.

Meanwhile, the control unit 150 may perform the role of controlling an overall operation of the system 100 for musculoskeletal disease prediction related to the present invention. The control unit 150 may perform a series of data processing for processing signals, data, information, and the like that are input or output through the constituent elements described above, or for providing or processing appropriate information and functions to a user.

Meanwhile, the present invention is intended to provide a method and system for deep learning-based musculoskeletal disease prediction. More specifically, the present invention is intended to provide the deep learning model 160 capable of minimizing an accident risk by accurately sensing positions and movements of objects such as a worker, work equipment, work tools, and a workpiece in a construction (or industrial) site, and hereinafter, a method for deep learning model-based musculoskeletal disease prediction will be described in more detail.

Meanwhile, in the present invention, a process of receiving an image of a worker serving as a photographing (or image capturing) target and a specific environment in which the worker is positioned may be performed (S310, see FIG. 2).

First, the control unit 150 may receive an image by distinguishing a worker serving as a photographing target and a specific environment in which the worker is positioned.

Here, an image of the worker may include various motions or behaviors performed by the worker in a workspace at a construction site. For example, behaviors such as the worker lifting an object above the head, a motion in which the worker bends or straightens the body, a motion in which the worker pushes or pulls an object, and the like may be included.

As illustrated in FIG. 3, in a construction site, various motions that may cause a musculoskeletal disease, such as a worker transporting a workpiece, using a ladder, or handling heavy tools, may be included. In addition, an image of a specific environment may include not only equipment such as a cart, lumber, a saw, a hammer, paint, a drill, a ladder, and a pickaxe, but also a workpiece and a structure.

In the present invention, a distinction between a worker and a specific environment in which the worker is positioned may be understood as one process performed to enable the deep learning model 160 to accurately detect objects in various situations.

Further, the control unit 150 may collect video (or image) data related to safety accidents at a construction site from various sources (for example, AI-Hub, a control system 1000, a database, web crawling, an API, a server linked to the system 100 for musculoskeletal disease prediction, an external server, etc.). For example, the control unit 150 may receive visual data including various classes, such as a worker, transport equipment, storage facilities, and work facilities within a construction site.

Here, data related to musculoskeletal diseases at a construction site may include at least one of an image of a worker serving as a photographing target and an image of a specific environment in which the worker is positioned, which have been performed above.

In this case, the image of the worker serving as a photographing target and the image of the specific environment in which the worker is positioned, which have been received, may be images taken from various angles to reflect various aspects in a construction site, and the control unit 150 may collect (or receive) images distinguished per object.

Meanwhile, the control unit 150 may process a received image as an input to the deep learning model 160.

The control unit 150 may apply various preprocessing techniques and augmentation to the received image to optimize performance of the deep learning model 160. In particular, in the present invention, in a data preprocessing process, data integrity may be ensured through removal of duplicate data, removal of outliers, and verification and correction of a bounding-box 450 and a keypoint(s) 550.

First, the control unit 150 may perform preprocessing on images of a construction site and images of working postures.

In one embodiment, the control unit 150 may improve data quality by identifying and removing images in which the same object is included multiple times among images of workers and images of a specific environment through removal of duplicate data in a preprocessing process.

In this case, the control unit 150 may remove noise of an image and may improve accuracy of object detection by using a Gaussian-blur filter and a Non-Local Means Denoising technique in the preprocessing process.

The Gaussian-blur filter is used to remove high-frequency components in an image and to reduce local noise. In particular, in a complex environment such as a construction site, background noise may interfere with object detection, and to solve this problem, when Gaussian blur is used, the noise may be smoothly processed, and boundaries of an object may be detected more clearly.

In addition, the Non-Local Means Denoising technique performs global noise removal of an image and is effective in reducing unnecessary background noise while preserving important object features. Through this, noise caused by various lighting conditions, shadows, or the like may be effectively removed.

Further, the control unit 150 may perform normalization on collected data so that feature values will have the same scale when the deep learning model 160 is trained, thereby preventing a bias toward specific values during a training process and enabling algorithms such as a gradient descent method to operate more effectively.

The control unit 150 may normalize pixel values included in images of a worker serving as a photographing target and images of a specific environment to a specific range (for example, a range of 0 to 1) to improve performance of the deep learning model 160. Normalization may ensure stability of learning and consistency of numerical calculations, thereby increasing a learning speed of the deep learning model 160 and improving performance.

Meanwhile, in the present invention, a process of specifying a first bounding-box corresponding to a worker and a second bounding-box corresponding to a specific environment from an image may be performed by using a pre-trained deep learning model (S320, see FIG. 2).

The bounding-box 450 is a rectangular region used to define a position of a specific object in an image or video. The bounding-box 450 is widely used in deep learning-based object detection or segmentation, and represents the bounds in which an object exists via coordinates and size.

The control unit 150 may define a boundary box (or a bounding-box 450) for an object in an image or video and may utilize the boundary box as training data of the deep learning model 160. Through this, the deep learning model 160 may learn features and patterns for distinguishing an object (a worker) and a background (a specific environment).

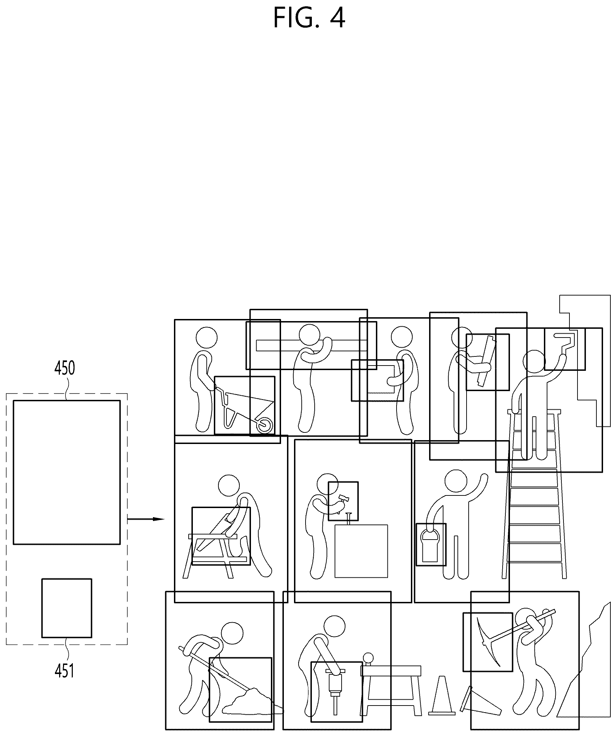

For example, as illustrated in FIG. 4, the control unit 150 may perform processing of a first bounding-box 450 corresponding to the worker included in a preprocessed image and a second bounding-box 451 corresponding to the work tool. In this case, in the present invention, bounding-boxes 450 and 451 processing for each of images of workers and work tools may be performed by using a preset annotation tool (e.g., ROBOFLOW™),

In the present invention, for convenience of description, the first bounding-box 450 corresponding to the worker and the second bounding-box 451 corresponding to the work tool will be collectively described as 'the bounding-box 450' without distinguishing between the two.

For example, the preset annotation tool provides a user-friendly interface and an automatic annotation function to enable a quick and accurate annotation task even for large-scale datasets. During performing an annotation task, quality of a dataset may be immediately checked through real-time feedback, and task speed may be greatly increased through automatic annotation utilizing a pre-trained model.

In addition, the control unit 150 may verify quality of annotated data by using a preset annotation tool. For example, in a data-quality verification process, the control unit 150 may perform verification on images of the worker and the specific environment, and when there exists a modification-target image in which the bounding-box 450 is incorrectly designated, the control unit 150 may modify the modification-target image by using the preset annotation tool.

The deep learning model 160 may receive image data of the worker and the specific environment as an input, may recognize a specific region (the worker and work tool) of the image, and may specify the bounding-box 450 surrounding the corresponding region by using the preset annotation tool.

As described above, an annotation tool used in the present invention provides support for (or supports) various formats, enabling data to be easily used in various learning frameworks such as a YOLOv8 model. In particular, in the present invention, labeling may be performed for each of a plurality of different objects (for example, a person, a cart, lumber, a ladder, a drill, a hammer, etc.), and the labeled data may be converted into a format that may be directly applied to learning of the deep learning model 160, thereby enabling efficient learning without a separate conversion process.

In addition, the annotation tool provides ease of use, automation functions, support for various formats, and integration with data-augmentation functions, enabling efficient annotation of large-scale datasets, real-time review of data quality, and quick correction of errors. This ensured that annotation task was performed consistently and accurately, and ultimately contributed to improving performance of the deep learning model 160.

Meanwhile, in the present invention, a process of extracting keypoints corresponding to a plurality of joints included in the body of a worker may be performed based on the first bounding-box (S330, see FIG. 2).

The first bounding-box 450 may be a bounding-box 450 corresponding to the worker.

The control unit 150 may identify joints included in the body of the worker and may define positions based on the first bounding-box 450. In this case, the control unit 150 may perform a process of finding approximate positions of joints by using the deep learning model 160 and specifying which part is a joint by recognizing a body structure of the worker. In this process, a region (ROI) inside the bounding-box 450 may be searched to identify the body structure of the worker, and an entire position range of major body parts (a head, shoulders, arms, legs, etc.) may be calculated. In addition, points within the bounding-box 450 that may represent joint positions may be detected and specified as candidate regions.

In addition, the control unit 150 may extract keypoints 550 for each of different joint positions specified inside the bounding-box 450. In this case, in the present invention, a keypoint 550 task may be performed by using a preset annotation tool (e.g., a ROBOFLOW tool) in the keypoint-extraction process.

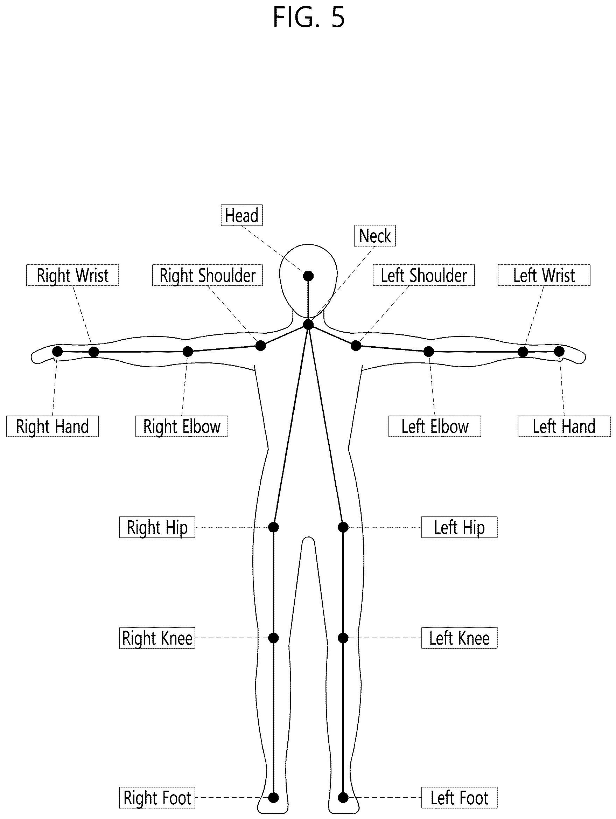

For example, as illustrated in FIG. 5, a human-skeleton structure composed of sixteen keypoints 550 including a head, a neck, a left shoulder, a right shoulder, a left elbow, a right elbow, a left wrist, a right wrist, a left hand, a right hand, a left hip, a right hip, a left knee, a right knee, a left foot, a right foot, and the like may be defined.

The control unit 150 may calculate precise positions based on joint candidates specified inside the bounding-box 450 by using the preset annotation tool and may adjust positions for each of the different joints to extract keypoints 550. In this process, when a specific keypoint 550 is not visible, the keypoint 550 may be displayed as being occluded by the bounding-box 450. When a keypoint 550 does not exist (for example, when the keypoint is not present in the object or is outside the image), the corresponding keypoint 550 may be deleted.

The control unit 150 may adjust positions corresponding to a plurality of joints included in the body of the worker based on the specified first bounding-box 450 and may extract keypoints for the adjusted joint positions in the form of (x, y) or (x, y, z) in a 2D or 3D coordinate system.

The control unit 150 may adjust scale, rotation, distortion, and the like through an image-alignment process so that each joint position of the body is accurately extracted.

For example, in a 2D image, when a worker's posture is tilted or distortion occurs due to a camera angle, the control unit 150 may allow joint positions to be adjusted to accurate positions inside the bounding-box 450. In addition, when sizes or positions of respective joints in an image are not uniform, the control unit 150 may allow the sizes or ratios to be adjusted to the same size or ratio at accurate positions inside the bounding-box 450. In addition, when the worker rotates in a specific direction, the control unit 150 may apply a rotation transformation based on relative positions of the joints.

For example, in a 3D image, the control unit 150 may convert and adjust relative positions between body joints into accurate coordinates by using the deep learning model 160. The deep learning model 160 may perform accurate position adjustment by learning movement ranges of joints and relationships between joints. Further, the control unit 150 may adjust distortion of an image or differences in distances between joints to fit the deep learning model 160 through a multi-point linear correction method or a geometric transformation.

A 2D image is a planar image composed of two dimensions (horizontal and vertical). All pixels are defined in fixed coordinates, and depth (z-axis) information is not included. A coordinate system may represent each pixel's position in the form of (x, y), in which horizontal (x) and vertical (y) positions are expressed.

A 3D image is composed of three dimensions (horizontal, vertical, and depth) and includes spatial depth (z-axis) information of an object. Through this, a position of an object may be defined within a real space. The coordinate system is in the form of (x, y, z), additionally including horizontal (x), vertical (y), and depth (z) information. In addition to pixels, information including spatial arrangement, distance, texture, and the like of each point may be included.

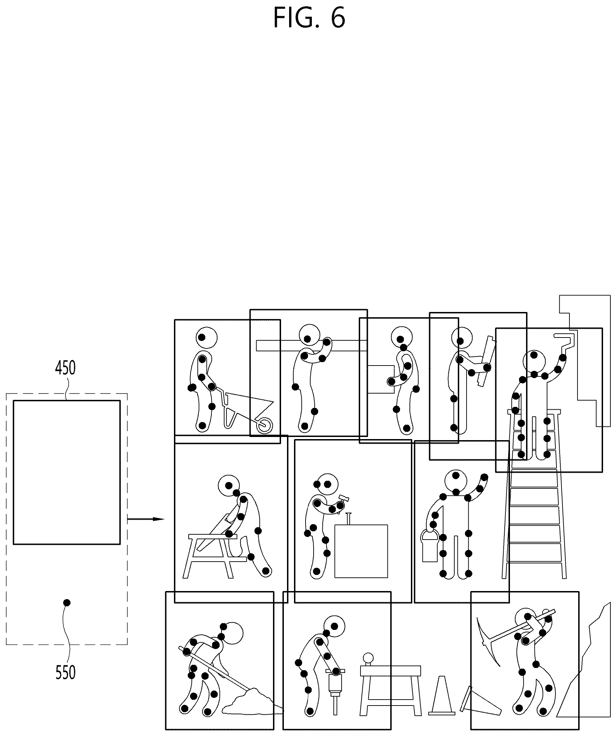

For example, as illustrated in FIG. 6, the keypoints 550 may be extracted such that joints positioned on the body of the worker may be expressed in a dot format. For example, sixteen major joints may be analyzed to extract the keypoints 550, and the keypoints 550 may express positions indicating the worker's joints in a dot format to concisely and quantitatively represent motion, and may be utilized for working-posture evaluation, injury-risk analysis, improvement of work efficiency, and the like.

After the keypoints 550 are extracted, the control unit 150 may perform data preprocessing and augmentation by using an annotation tool (e.g., ROBOFLOW™) to increase an amount and diversity of a dataset. For example, in a data-preprocessing step, the control unit 150 may scale-adjust all images of the dataset to 640 × 640 pixels and may apply contrast stretching to automatically adjust image contrast. In this case, an image may be made sharper and detailed information may be improved, enabling image analysis and processing.

The annotation tool is integrated with data-augmentation functions, enabling augmentation of a dataset in various situations after annotation task so that a (deep learning) model may learn more effectively in diverse environments. Due to such integrated functions, annotation and augmentation processes may be handled on a single platform, greatly improving work efficiency.

Further, the annotation tool may continuously improve accuracy and reliability of a dataset by providing an environment in which annotation errors may be easily corrected. That is, in the present invention, by utilizing annotation functions of the annotation tool and data-quality management tools, accurate and diverse datasets for development of the deep learning model 160 for predicting musculoskeletal diseases in a construction site may be effectively constructed.

Meanwhile, in the present invention, after a data-preprocessing and image-annotation process, images may be augmented. In an image-augmentation process, a series of transformations may be applied to an original image to augment size, diversity, and types of a dataset, enabling effective data augmentation without new data collection. Here, data used for augmentation may be at least one of an image before a bounding-box 450 and a keypoint 550 are assigned and/or an image to which the bounding-box 450 and the keypoint 550 are assigned. In the present invention, augmentation-target data is not limited to any one of the above.

The control unit 150 may build a training dataset by augmenting images (or annotated images) of the worker serving as a photographing target and a specific environment in which the worker is positioned by using a preset augmentation technique. Here, the preset augmentation technique may be a technique selected to transform data so that various motions of a worker and environmental conditions may be better reflected.

Meanwhile, in the present invention, by using extracted keypoints, a process of calculating angles between a plurality of joints and calculating a risk score on calculated angles between the plurality of joints may be performed (S340, see FIG. 2).

The control unit 150 may manage a flow of data and may calculate angles based on keypoint 550 coordinates. For example, extracted keypoints 550 may be expressed as coordinates in the form of (x, y). The control unit 150 may calculate vectors between keypoints 550 and may mathematically define the movements of the joints.

The control unit 150 may project the positions of the extracted keypoints 550 onto a 2D plane (image coordinates) to simplify joint calculation. Through this, a joint angle may be calculated from a 2D image even without 3D data. For example, the joint angle may be calculated by using inverse trigonometric functions.

For example, the control unit 150 may define vectors by using coordinates of two adjacent joints connected around positions of respective keypoints 550. For example, as illustrated in FIG. 7, (a) shows an angle when an arm is bent based on keypoints 550 positioned at a shoulder 711, an elbow 712, and a wrist 713, and (b) shows an angle when an arm is stretched based on keypoints 550 positioned at the shoulder 711, the elbow 712, and the wrist 713. The shoulder 711, the elbow 712, and the wrist 713 are one example of adjacent joints, and various joints may be selected.

The control unit 150 may define coordinates such as a keypoint coordinate (x1, y1) for the shoulder 711, a keypoint coordinate (x2, y2) for the elbow 712, and a keypoint coordinate (x3, y3) for the wrist 713. In this process, vectors between respective keypoints 550 may be defined as differences between two points. For example, a first vector (elbow 712 - shoulder 711, v1) and a second vector (elbow 712 - wrist 713, v2) may be defined. In this case, the control unit 150 may calculate magnitudes of the first vector (v1) = (x2 - x1, y2 - y1) and the second vector (v2) = (x3 - x2, y3 - y2) by using [Equation 1] below.

[Equation 1]

$$|v_1| = \sqrt{(x_2 - x_1)^2 + (y_2 - y_1)^2}$$

$$|v_2| = \sqrt{(x_3 - x_2)^2 + (y_3 - y_2)^2}$$

Further, the control unit 150 may calculate a magnitude of a joint angle ($A_i$) by using [Equation 2] below.

[Equation 2]

$$A_i = \arccos\left(\frac{v_1 \times v_2}{(|v_1| \times |v_2|)}\right) \times \left(\frac{180}{\pi}\right)$$

Meanwhile, the system 100 for musculoskeletal disease prediction according to the present invention may include a joint-angle calculation algorithm. The joint-angle calculation algorithm may be an algorithm that outputs an angle when a keypoint 550 is input. Specifically, this process may be performed through the control unit 150, and the control unit 150 may store and manage extracted keypoint coordinates. The indices of three keypoints 550 necessary for calculating an angle may be defined. For example, a first index (x1, y1), a second index (x2, y2), and a third index (x3, y3) may be designated. In this case, the control unit 150 may obtain a first vector ((x1, y1), (x2, y2)) and a second vector ((x1, y1), (x3, y3)) as vectors for the first index (x1, y1), the second index (x2, y2), and the third index (x3, y3) by using a preset function. Next, an angle between the first vector and the second vector may be output by using the preset function. The joint-angle calculation algorithm according to the present invention may be expressed as follows.

---

**Joint angle calculation algorithm**

---

Input: keypoints

Output: angle

1. Get the keypoints of the detected object results

    first_detection_keypoints = results[0].keypoints.xy

2. Get the keypoint coordinates

    keypoints = first_detection_keypoints[0]

3. Define the indices of the keypoints to calculate the angle

    index_1 = a

    index_2 = b

    index_3 = c

4. Get the coordinates of the three keypoints

    (x1, y1) = keypoints[index_1]

    (x2, y2) = keypoints[index_2]

    (x3, y3) = keypoints[index_3]

5. Calculate vectors

    Define function calculate_vector(p1, p2):

        return (p2[0] - p1[0], p2[1] - p1[1])

    v1 = calculate_vector((x1, y1), (x2, y2))

    v2 = calculate_vector((x1, y1), (x3, y3))

6. Calculate the angle between the two vectors

    Define function calculate_angle(v1, v2):

        - Calculate dot product: dot_product = v1[0] * v2[0] + v1[1] * v2[1]

        - Calculate vector lengths:

            v1_norm = sqrt(v1[0]^2 + v1[1]^2)

            v2_norm = sqrt(v2[0]^2 + v2[1]^2)

        - Calculate cos(theta): cos_theta = dot_product / (v1_norm * v2_norm)

        - Limit cos_theta to the range [-1, 1] to handle numerical errors

        - Calculate the angle in radians: angle_rad = acos(cos_theta)

---

        - Convert to degrees: angle_deg = degrees(angle_rad)

        - Return angle_deg

    angle_deg = calculate_angle(v1, v2)

7. Print the calculated result

    Print "angle: " – angle_deg

---

In addition, the control unit 150 may convert angles between the calculated plurality of joints into a form in which the angles may be analyzed and processed by the deep learning model 160, and may input the converted data into the model.

The control unit 150 may vectorize and normalize the calculated angles between joints into a certain range (e.g., 0, 1) so as to fit an input format of the deep learning model 160. For example, when a maximum angle is 180 degrees, 90 degrees may be converted into 0.5, and 45 degrees may be converted into 0.25. In this process, when an angle between the joints is missing (e.g., when a keypoint is not accurately extracted), the missing value may be processed. In this case, the control unit 150 may replace the missing value with an average value of the corresponding joint angle and may replace the missing value with 0.

The control unit 150 may unify a size of input data so that the input data may be matched to the deep learning model 160. For example, when a model input size is N = 20, a number of joint-angle data may be adjusted to 20. When the number is insufficient, insufficient portions may be supplemented to satisfy the data size, and when the number exceeds the size, unnecessary data may be excluded to adjust the data to an appropriate size.

In addition, the control unit 150 may analyze input joint-angle data by using the deep learning model 160 and may calculate a risk score 800 related to a working posture. The risk score 800 may be used as an index for evaluating stability, efficiency, and possibility of injury of a working posture.

In addition, the deep learning model 160 may have a structure capable of performing working-posture and risk-score 800 calculation. The deep learning model 160 may analyze a pattern of a working posture and a risk factor by delivering the input data to multiple layers.

Specifically, the deep learning model 160 may process the input data to extract features related to the working posture, may perform calculation for evaluating risk of the posture based on the feature data, and may generate a regression or classification result for calculating the risk score 800.

For example, the deep learning model 160 may be designed to calculate the risk score 800 as a value between 0 and 7. The deep learning model 160 may be designed to analyze a risk factor of a working posture based on the input data and finally to calculate the risk score 800 by utilizing extracted features and joint angles.

Meanwhile, in the present invention, a process of specifying a risk degree for a worker based on a calculated risk score may be performed (S350, see FIG. 2).

The deep learning model 160 may receive, as input, the obtained risk score 800, may specify the risk degree of the worker, and may evaluate a state of the worker. The risk-degree specification may quantitatively evaluate stability of a working posture and possibility of injury.

The deep learning model 160 may specify a first risk degree, a second risk degree, and a third risk degree according to a preset criterion based on the input risk score 800.

For example, the preset criterion may be designated based on a range corresponding to the second risk degree.

The deep learning model 160 may determine that a working posture includes a risk factor and that there is a possibility of injury, and, for example, when a risk score is a value between 3 and 4, the risk score may be specified as the second risk degree (medium risk).

The deep learning model 160 may determine that a working posture is stable and that there is almost no risk of injury, and, for example, when a risk score is a value greater than or equal to 0 and less than 3, the risk score may be specified as the first risk degree (low risk).

The deep learning model 160 may determine that a working posture is very dangerous and that immediate improvement is required, and, for example, when a risk score is a value greater than 4 and less than or equal to 7, the risk score may be specified as the third risk degree (high risk).

Further, the control unit 150 may flexibly select and calculate joint angles according to various work environments, and may specify a risk degree based on the joint angles. In this case, the control unit 150 may help identify a highly risky posture by analyzing angle changes of different joints, and may provide response action information such as risk alerts and improvement recommendations.

Meanwhile, in the present invention, a process of generating response action information according to the specified risk degree may be performed (S360, see FIG. 2).

The control unit 150 may generate response action information to ensure safety of a worker and to reduce a possibility of injury based on the specified first risk degree (low risk), second risk degree (medium risk), and third risk degree (high risk). The control unit 150 may provide specific response action information according to the specified risk degree to improve an environment and to increase efficiency and safety of the worker.

Action information generated according to the respective specified risk degrees in the present invention may be different.

Different response action information, matched to the respective specified risk degrees, may exist. Here, the different response action information may include first response action information matched to the first risk degree, second response action information matched to the second risk degree, and third response action information matched to the third risk degree.

For example, the different response action information may include at least one among i) working-posture monitoring and warning, provision of basic training, work-time adjustment, environment adjustment, and maintenance of work records, ii) real-time correction guidance, recommendation of equipment use, improvement of a work environment, additional safety training, recommendation of work suspension, and limitation of repetitive work, iii) immediate warning and work suspension, provision of a working-posture correction program, notification of a possibility of injury, equipment support, checking of a worker's health condition, and periodic monitoring, iv) temperature, humidity, and lighting adjustment, wearing of safety equipment, checking of equipment conditions, and marking of risk zones, v) deep learning-based real-time warning, provision of AR/VR simulation, and data-based improvements, vi) optimization of work processes, strengthening of teamwork, worker education programs, and updating of work regulations, and vii) emergency medical support, an accident reporting system, work stoppage, and evaluation. However, the information included in the different action information is not necessarily limited thereto, and various actions other than those mentioned above may be further included.

In this case, actions performed according to the first response action information, the second response action information, and the third response action information may differ from one another.

For example, as illustrated in FIG. 8, the control unit 150 may generate an appropriate warning phrase 810 and a notification based on the risk score 800. (a) illustrates a case in which a workpiece is being lifted in a posture that places a burden on the waist. In this case, a notification may be provided together with a warning phrase 810 stating, "Please straighten your back and bend your knees to maintain a correct posture." Here, the notification may be provided as a warning sound together with a warning display to inform the worker of a dangerous condition.

The control unit 150 may visually guide the worker to a corrected posture 850 proposed by the deep learning model 160. In addition, the control unit 150 may generate an explanation phrase 820 emphasizing importance of a correct working posture together with the corrected posture. For example, (b) illustrates a case in which the corrected posture is notified as an image. In this case, together with the corrected posture image 850, a phrase 820 stating, "This posture may prevent back injuries." may be provided. The control unit 150 may generate a 3D-simulation image or animation of the corrected posture to help the worker intuitively understand the posture, and may enable the worker to easily understand the posture by comparing the corrected posture with a current posture by using an image-processing technology.

The control unit 150 may provide learning feedback to the deep learning model 160 based on data stored in the storage unit 140.

In addition, the control unit 150 may continuously track a work state of the worker and may provide an immediate warning when a new dangerous situation occurs. For example, a dangerous situation may be a situation in which an improper posture or a burden on the body due to a workpiece or a work tool occurs.

Further, the control unit 150 may generate correction information in a personalized manner according to a work pattern of the worker and data previously stored in the storage unit 140. For example, more detailed corrected-posture guidance may be provided to a beginner worker, and concise warnings and correction phrases may be provided to a skilled worker.

Furthermore, the control unit 150 may propose whether to adjust a work environment. For example, the control unit 150 may propose whether to adjust a worktable height, equipment arrangement, and use of auxiliary equipment.

As described above, the method and system for deep learning-based musculoskeletal disease prediction according to the present invention may collect images of a worker serving as a photographing target and of a work environment and may provide an analysis result thereof. Data collection may be performed only with a camera device, thereby reducing initial installation and maintenance costs compared to sensor-based systems.

Further, the method and system for deep learning-based musculoskeletal disease prediction according to the present invention may detect joint positions by using the deep learning model 160 and may analyze a working posture by calculating angles between joints. Through this, joint positions may be detected only from images without attaching sensors or using additional equipment, and even when a worker changes a posture or performs complex motions, joint positions may be tracked in real time.

Further, the method and system for deep learning-based musculoskeletal disease prediction according to the present invention may provide warnings and improvement guidance by using a joint angle as an input. Through this, a possibility of occurrence of a musculoskeletal disease may be identified at an early stage, and preventive actions may be taken.

Further, the method and system for deep learning-based musculoskeletal disease prediction according to the present invention may predict a risky posture at an early stage and may support a worker to recognize the posture in real time. Through this, a worker may immediately correct a risky posture and may maintain a safe posture, thereby preventing occurrence of a musculoskeletal disease.

Meanwhile, the present invention described above may be executed by one or more processes on a computer and implemented as a program that may be stored on a computer-readable medium (or recording medium).

Further, the present invention described above may be implemented as computer-readable code or instructions on a medium in which a program is recorded. That is, the present invention may be provided in the form of a program.

Meanwhile, the computer-readable medium includes all kinds of recording devices for storing data readable by a computer system. Examples of computer-readable media include hard disk drives (HDDs), solid state disks (SSDs), silicon disk drives (SDDs), ROMs, RAMs, CD-ROMs, magnetic tapes, floppy discs, optical data storage devices, and the like.

Further, the computer-readable medium may be a server or cloud storage that includes storage and that the electronic device is accessible through communication. In this case, the computer may download the program according to the present invention from the server or cloud storage, through wired or wireless communication.

Further, in the present invention, the computer described above is an electronic device equipped with a processor, that is, a central processing unit (CPU), and is not particularly limited to any type.

Meanwhile, it should be appreciated that the detailed description is interpreted as being illustrative in every sense, not restrictive. The scope of the present invention should be determined on the basis of the reasonable interpretation of the appended claims, and all of the alternations within the equivalent scope of the present invention belong to the scope of the present invention.

**Claims**

1. A method for deep learning-based musculoskeletal disease prediction, the method comprising:

   receiving an image of a worker serving as a photographing target and a specific environment in which the worker is positioned;

   specifying, from the image, a first bounding-box corresponding to the worker and a second bounding-box corresponding to the specific environment by using a pre-trained deep learning model;

   extracting keypoints corresponding to a plurality of joints included in a body of the worker based on the first bounding-box;

   calculating angles between the plurality of joints by using the extracted keypoints and calculating a risk score on the calculated angles between the plurality of joints;

   specifying a risk degree for the worker based on the calculated risk score; and

   generating response action information according to the specified risk degree.

2. The method of claim 1, wherein the image includes at least one among the worker's posture, motion, motion pattern, a body of the worker, and the specific environment, and

   wherein the specific environment includes at least one among a workspace in which the worker is working and positions of tools, equipment, and a workpiece positioned in the workspace.

3. The method of claim 2, wherein the specifying of the bounding-box comprises:

   processing the received image as an input to the deep learning model;

   analyzing, by the deep learning model, the worker and the specific environment from the image; and

   specifying the first bounding-box corresponding to the worker and the second bounding-box corresponding to the specific environment by using a result of the analysis.

4. The method of claim 3, wherein the extracting of the keypoints comprises:

   adjusting joint positions corresponding to the plurality of joints according to a body structure of the worker based on the first bounding-box; and

   extracting the keypoints from the joint positions adjusted according to the body structure.

5. The method of claim 4, wherein the joint-angle calculation comprises:

   generating vectors corresponding to the joint positions based on the extracted keypoints; and

   calculating joint angles between the joint positions by using the generated vectors.

6. The method of claim 5, wherein the risk-score calculation comprises:

   predicting a risk level of the worker by using the calculated joint angles; and

   calculating the risk score for the joint angles based on a result of the prediction.

7. The method of claim 6, wherein the specifying of the risk degree comprises:

   processing the risk score on the calculated joint angles as an input to the deep learning model; and

   specifying, by the deep learning model, the risk degree corresponding to the risk score among a plurality of risk degrees based on a preset criterion, and

   wherein the plurality of risk degrees include at least one among a first risk degree specified based on the risk score not satisfying the preset criterion, a second risk degree specified based on the risk score satisfying the preset criterion, and a third risk degree specified based on the risk score exceeding the preset criterion.

8. The method of claim 7, wherein different response action information exist matched to the respective plurality of risk degrees, and the different response action information include at least one among first response action information matched to the first risk degree, second response action information matched to the second risk degree, and third response action information matched to the third risk degree, and

wherein the generating of the response action information comprises:

generating the second response action information or the third response action information for predicting a musculoskeletal disease, when the specified risk degree corresponds to at least one of the second risk degree and the third risk degree that are different from the first risk degree.

9. A system for deep learning-based musculoskeletal disease prediction, the system comprising:

a data collection unit configured to receive an image of a worker serving as a photographing target and a specific environment in which the worker is positioned; and

a control unit configured to specify a first bounding-box corresponding to the worker and a second bounding-box corresponding to the specific environment from the image by using a pre-trained deep learning model,

wherein the control unit is configured to:

extract keypoints corresponding to a plurality of joints included in a body of the worker based on the first bounding-box;

calculate angles between the plurality of joints by using the extracted keypoints and calculate a risk score on the calculated angles between the plurality of joints;

specify a risk degree for the worker based on the calculated risk score; and

generate response action information according to the specified risk degree.

10. A program stored on a computer-readable recording medium, executed by one or more processes in an electronic device, the program comprising instructions for performing:

receiving an image of a worker serving as a photographing target and a specific environment in which the worker is positioned;

specifying, from the image, a first bounding-box corresponding to the worker and a second bounding-box corresponding to the specific environment by using a pre-trained deep learning model;

extracting keypoints corresponding to a plurality of joints included in a body of the worker based on the first bounding-box;

calculating angles between the plurality of joints by using the extracted keypoints and calculating a risk score on the calculated angles between the plurality of joints;

specifying a risk degree for the worker based on the calculated risk score; and

generating response action information according to the specified risk degree.

FIG. 1

SYSTEM FOR DEEP LEARNING-BASED MUSCULOSKELETAL DISEASE PREDICTION — 100

DATA COLLECTION UNIT — 110

DATA PROCESSING UNIT — 120

COMMUNICATION UNIT — 130

STORAGE UNIT — 140

CONTROL UNIT — 150

DEEP LEARNING MODEL — 160

# FIG. 2

START

RECEIVE IMAGE OF WORKER SERVING
AS PHOTOGRAPHING TARGET AND SPECIFIC ENVIRONMENT
IN WHICH WORKER IS POSITIONED ——S310

SPECIFY FIRST BOUNDING-BOX
CORRESPONDING TO WORKER AND SECOND BOUNDING-BOX
CORRESPONDING TO SPECIFIC ENVIRONMENT FROM IMAGE
BY USING PRE-TRAINED DEEP LEARNING MODEL ——S320

EXTRACT KEYPOINTS CORRESPONDING TO
PLURALITY OF JOINTS INCLUDED IN BODY OF WORKER
BASED ON FIRST BOUNDING-BOX ——S330

CALCULATE ANGLES BETWEEN PLURALITY OF JOINTS
BY USING EXTRACTED KEYPOINTS AND CALCULATE RISK SCORE
ON CALCULATED ANGLES BETWEEN PLURALITY OF JOINTS ——S340

SPECIFY RISK DEGREE FOR WORKER
BASED ON CALCULATED RISK SCORE ——S350

GENERATE RESPONSE ACTION INFORMATION
ACCORDING TO SPECIFIED RISK DEGREE ——S360

END

# FIG. 3

FIG. 4

450

451

# FIG. 5

# FIG. 6

FIG. 7

711

713

712

(a)

711

713

712

(b)

# FIG. 8

(a)

(b)

Europäisches
Patentamt

European
Patent Office

Office européen
des brevets

# EUROPEAN SEARCH REPORT

**Application Number**

EP 25 21 9651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/327465 A1 (BAEK STEPHEN [US] ET AL) 15 October 2020 (2020-10-15) * paragraph [0002] - paragraph [0104] * ----- | 1-10 | INV. G06V10/46 G06V10/62 G06V10/25 |
| A | WANG CHEN ET AL: "Real-time ergonomic risk assessment in construction using a co-learning-powered 3D human pose estimation model", COMPUTER-AIDED CIVIL AND INFRASTRUCTURE ENGINEERING, BLACKWELL PUBLISHERS, MALDEN, US, vol. 39, no. 9, 18 December 2023 (2023-12-18), pages 1337-1353, XP072621580, ISSN: 1093-9687, DOI: 10.1111/MICE.13139 * sections 2-5 * ----- | 1-10 | G06V10/44 G06V10/764 G06V10/774 G06V10/82 G06V20/20 G06V20/60 G06V40/10 G06V40/20 |

TECHNICAL FIELDS
SEARCHED     (IPC)

G06V

The present search report has been drawn up for all claims

| Place of search | Date of completion of the search | Examiner |
|---|---|---|
| The Hague | 20 February 2026 | Atmatzidis, Lazaros |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 21 9651

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2026

| Patent document cited in search report | Publication date | Patent family member(s) | Publication date |
|---|---|---|---|
| US 2020327465 A1 | 15-10-2020 | AU 2020270998 A1 | 02-12-2021 |
| | | CA 3136732 A1 | 15-10-2020 |
| | | CN 113874912 A | 31-12-2021 |
| | | EP 3953901 A1 | 16-02-2022 |
| | | KR 20220044677 A | 11-04-2022 |
| | | SG 11202111352X A | 29-11-2021 |
| | | US 2020327465 A1 | 15-10-2020 |
| | | US 2022237537 A1 | 28-07-2022 |
| | | US 2024161039 A1 | 16-05-2024 |
| | | WO 2020210004 A1 | 15-10-2020 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240178492